# EUROPEAN PATENT SPECIFICATION

(11) **EP 2 158 907 B1**
(45) Date of publication and mention of the grant of the patent: **05.08.2015**
(21) Application number: 09014985.7
(22) Date of filing: 17.09.1998
(51) Int. Cl.: A61K 8/02, A61K 31/198, A61P 43/00, A61K 8/14, A61K 8/44

(54) **TOPICAL ADMINISTRATION OF ARGININE FOR PAIN TREATMENT**
Topische Arginin-Verabreichung zur Schmerzbehandlung
Administration topicale d'arginine pour le traitement de la douleur

(30) Priority: 17.09.1997 US 932227; 17.09.1997 US 932595; 17.09.1997 US 936188; 17.09.1997 US 936189
(43) Date of publication of application: 03.03.2010
(62) Divisional of application: 98946099.3
(73) Proprietor: Strategic Science & Technologies, LLC, Cambridge MA 02141 (US)
(72) Inventor: Fossel, Eric, Cambridge, Massachusetts 02139 (US)
(74) Representative: Heare, Tanya

(56) References cited:
- EP-A2- 0 424 028
- WO-A1-97/10830
- FR-A- 2 602 678
- US-A- 5 595 753
- PATENT ABSTRACTS OF JAPAN vol. 016, no. 148 (C-928), 13 April 1992 (1992-04-13) -& JP 04 005231 A (MORISHITA PHARMACEUT CO LTD; OTHERS: 01), 9 January 1992 (1992-01-09)

## Description

### Background of the Invention

### Field of the Invention

This invention relates, in general, to a delivery vehicle, for topical application to the skin, which contains substances including, but not limited to, L-arginine and/or salts and/or derivatives thereof. The purpose of this delivery vehicle is to introduce L-arginine into human or mammalian tissue for the purpose of producing beneficial effects such as the relief of pain. The scope of the invention is limited by the appended claims.

### Prior Art

Approaches to improving local blood flow have been many and consist of both systemic and topical approaches. Many beneficial effects could be obtained should improvement in local blood flow be achieved since impairment of local blood flow causes a variety of negative consequences.

There have been many approaches to overcoming pain in the prior art. These attempts consist mostly of oral analgesic agents ranging from aspirin and ibuprofen to more powerful narcotic oral agents such as codeine. Alternatively, in cases where a subject suffers from severe pain, narcotic agents including morphine have been used. It has been found that the amino acid L-arginine is a precursor to the natural endogenous analgesic substance, kyotorphin. It has been shown that intravenous administration of large amounts (30 g/patient) of L-arginine is successful in overcoming pain. It is thought that this treatment exerts its effect by increasing levels of kyotorphin. However, this treatment is impractical for use in everyday life and is reserved only for the most extreme forms of chronic pain. Others have found that nitric oxide, whose biochemical precursor is L-arginine, potentiates b-endorphin-induced pain relief. Another form of pain relief, which is distinct from the use of arginine, is the application of capsaicin, a substance derived from hot peppers.

US 5,595,753 discloses topical formulations and methods for treating hemorrhoidal pain and sphincter and smooth muscle spasm in the gastrointestinal tract.

### SUMMARY OF THE INVENTION

In accordance with the present invention, it has been discovered that the delivery of the nitric oxide precursor, L-arginine and its claimed derivatives, by a topical approach, produces a variety of beneficial effects due to the increased blood flow caused by the subsequent release of nitric oxide into the blood. These beneficial effects include the treatment of pain. Also, according to the present invention, the topical delivery of L-arginine, when fortified with capsaicin, capsicum or its source extract, oleoresin capsicum, can alleviate pain when administered to a specific area of the body.

In a first aspect, therefore, the invention provides a composition for use as a medicament for topical application to skin for treating pain, the composition comprising: a nitric oxide releasing substance selected from a member of the group consisting of L-arginine, L-arginine salts and L-arginine derivatives selected from the group consisting of ethyl, methyl, propyl, isopropyl, butyl, isobutyl, or t-butyl esters of L-arginine and salts thereof; and an ionic salt, wherein the ionic salt has an ionic strength of at least two times the physiological ionic strength of blood.

In an embodiment, the present invention provides a composition of the first aspect comprising sodium chloride or other salts at a concentration sufficient to produce a hostile biophysical environment and capsaicin or oleoresin capsicum in concentrations sufficient to produce the desired effect, in a topical form, for application directly to the painful area to overcome pain.

### OBJECTS OF THE PRESENT INVENTION

Accordingly, an object of the present invention is to relieve pain by means of increasing local blood flow through the use of a nitric oxide releasing substance.

### DETAILED DESCRIPTION OF THE PREFERRED EMBODIMENTS

At the outset, it is to be understood that the invention is described in its broadest overall aspects with a more detailed description following. The present invention in one embodiment is a composition for delivery of L-arginine or its derivatives to cause beneficial effects by its release of nitric oxide. The present invention relies on the discovery that a carrier or vehicle for arginine will expel the arginine if it contains, in addition to the arginine, an agent which causes the arginine to leave the carrier and enter the tissue.

### THE TOPICAL DELIVERY VEHICLE

One embodiment of the present invention comprises a topical delivery vehicle having properties of excellent absorption into the skin. This topical delivery vehicle contains L-arginine hydrochloride (12.5% w/v), choline chloride (10%), sodium chloride (5% w/v) and magnesium chloride (5% w/v). As used herein, all expressions of concentration using the designation "%w/v" shall mean percent weight per total volume of the preparation regardless of form, e.g., cream, tablet, liquid, unless specified otherwise.

The components of the base cream may be those commonly found in hand creams, such as water (20-80%w/v), mineral oil (3-18%w/v), glyceryl stearate (0.5-12%w/v), squalene(0.2-12%w/v), cetyl alcohol (0.1-11%w/v), propylene glycol stearate (0.1-11%w/v), wheat germ oil (0.1-6%w/v), glyceryl stearate (0.1-6%w/v), isopropyl myristate (0.1-6%w/v), stearyl stearate (0.1-6%w/v), polysorbate 60 (0.1-5%w/v), propylene glycol (0.05-5%w/v), tocopherol acetate (0.05-5%), collagen (0.05-5%), sorbitan stearate (0.05-5%), vitamin A&D (0.02-4%w/v), triethanolamine (0.01-4%w/v), methylparaben (0.01-4%w/v), aloe vera extract (0.01-4%w/v), imidazolidinyl urea (0.01-4%w/v), propylparaben (0.01-4%w/v), bha (0.01-4%w/v), L-arginine hydrochloride (0.25% to 25%w/v), sodium chloride (0.25% to 25%w/v), magnesium chloride (0.25% to 25%w/v).

L-arginine hydrochloride provides a precursor to the molecule, nitric oxide, NO. Nitric oxide is the substance that relaxes the blood vessels, allowing for increased blood flow. The concentration of the L-arginine-based compound, e.g., L-arginine hydrochloride, is preferably about between 0.25 to 25%w/v.

Choline chloride, sodium chloride and magnesium chloride are non-limiting examples of salts which provide a high ionic strength environment for the highly charged molecule, L-arginine. This high ionic strength environment is an example of a hostile biophysical environment for L-arginine. That is, the highly charged ionic strength provided by the salts to the L-arginine carrier is an unfavorable environment for the highly charged L-arginine which facilitates or promotes L-arginine migration out of the carrier and into a more hospitable, less charged environment such as human tissue. The ionic strength is preferably any amount greater than two times the physiological ionic strength of blood.

Thus, the topical delivery vehicle containing the nitric oxide releasing substance, choline chloride, sodium chloride and/or magnesium chloride is the agent which produces beneficial effects. Another important embodiment of the present invention consists of a topical delivery vehicle as described above, wherein the vehicle also includes capsaicin (0.025%w/v) or oleoresin capsicum (0.5%w/v). The purpose of the capsaicin or oleoresin capsicum is to deplete sensory fibers of substance P (SP). The cream is the agent which is applied to human or mammalian tissue in order to aid in overcoming pain.

The treatment consists of applying the cream directly to the painful area. When carried out every four hours for a period of 12-16 hrs and then maintained with twice daily administration substantial relief from pain results.

### OTHER ACTIVE INGREDIENTS

While L-arginine hydrochloride is the preferred active agent for use as a nitric oxide releasing substance other agents could be used which are also precursors or donors of nitric oxide. Specifically, L-arginine hydrochloride is preferred due to the fact that it is a naturally occurring compound that is nontoxic, highly soluble and inexpensive. Other precursors which may be used include D,L -arginine, L-arginine, alkyl (ethyl, methyl, propyl, isopropyl, butyl, isobutyl, t-butyl) esters of L-arginine and salts thereof. Pharmaceutically acceptable salts include hydrochloride, glutamate, butyrate, and glycolate.

In the case of an alternative active agent being used it would be simply substituted for L-arginine in a delivery preparation and the preparation used as in the case of the L-arginine preparation. The cream may contain capsaicin or oleoresin capsicum in addition to L-arginine.

### OTHER MEANS OF EFFECTING ABSORPTION

A variety of carriers for effecting absorption are possible. One approach to effectuate the absorption of a highly charged molecule such as L-arginine into tissue is to either create a biophysically hostile environment in the delivery vehicle such that L-arginine would prefer to be in tissue. Other approaches include packaging L-arginine in such a way that it is carried into tissue and/or neutralize its charge by derivatization or forming a neutral salt. Examples of biophysically hostile environments include but are not limited to, high ionic strength, high or low pH and/or highly hydrophobic environments. Examples of packaging which would be carried into tissue include liposomes or emulsions of collagen, collagen peptides or other components of skin or basement membrane. An example of neutralization of charge include the salt, arginine glutamate, which is electronically neutral. Other acceptable arginine salts are set forth herein above.

In each case of creating a hostile biophysical environment for the active agent, the agent was added to an appropriate preparation. In the case of creating a high ionic strength ion environment, salts such as but not limited to sodium chloride, potassium chloride, choline chloride, lithium chloride, alone or in combination were added in high concentration to achieve an ionic strength greater than two times the physiological strength of blood. Other highly charged molecules such as polylysine, polyglutamine, polyaspartate or copolymers of such charged amino acids may be used to create the hostile biophysical environment.

Alternatively a hostile biophysical environment may be created by placing the highly charged L-arginine in an hydrophobic, oily environment such as in an oil-based cream containing little or no water. The preferred hydrophobicity or rho (p) is greater than two times the physiological hydrophobicity of blood. Absorption may further be aided by combining the use of hostile biophysical environments with the use of penetrating agents such as oleoresin capsicum or its constituents or molecules containing heterocyclic rings to which are attached hydrocarbon chains.

If a high or low pH environment is the hostile environment chosen, the preferred pH range is about between 3 to 11 pH.

### CLINICAL APPLICATIONS

### Example 1

In a 52 year old woman with a 13 year history of chronic neck pain, administration of a penetrating cream containing L-arginine hydrochloride (12.5%w/v), choline chloride (10%w/v), magnesium chloride (5%w/v) and sodium chloride (5%w/v) every four hours for 1 day followed by twice daily administration directly to the neck brought relief from the pain within the first day. This relief of symptoms was maintained by continuation of the twice daily treatment.

### Example 2

In a 35 year old man with a three year history of shoulder pain, application of a penetrating cream containing L-arginine hydrochloride (12.5%w/v) and choline chloride (10%w/v), magnesium chloride (5%), sodium chloride (5%) and oleoresin capsicum (0.5%) every four hours for 1 day followed by twice daily applications directly to the painful area brought relief from pain within 8 hours. The relief of symptoms was maintained by continuation of twice daily treatment regimen.

As illustrated by the examples, it can be seen that the present invention provides a composition comprising a nitric oxide releasing substance in a delivery vehicle which, when applied to a person with pain, causes a reduction or cessation of pain by use of the body's own mechanisms. This effect is achieved by providing at the local site, the biochemical substrate from which the controlling substance, L-arginine, causes enhancement in levels of the natural analgesic kyotorphin and/or enhancement of the effectiveness of natural endorphins. Further, it is seen in the present invention when L-arginine is used in conjunction with capsaicin or oleoresin capsicum, an additional mechanism of pain relief, depletion of substance P from sensory fibers is activated.

## Claims

1. A composition for use as a medicament for topical application to skin for treating pain, the composition comprising:
a nitric oxide releasing substance selected from a member of the group consisting of L-arginine, L-arginine salts and L-arginine derivatives selected from the group consisting of ethyl, methyl, propyl, isopropyl, butyl, isobutyl, or t-butyl esters of L-arginine and salts thereof; and
an ionic salt, wherein the ionic salt has an ionic strength of at least two times the physiological ionic strength of blood.

2. A composition for use as claimed in claim 1, **characterised in that** the composition further comprises capsaicin.

3. A composition for use as claimed in claim 1, **characterised in that** the composition further comprises oleoresin capsicum.

4. A composition for use as claimed in any previous claim, **characterised in that** the ionic salt comprises sodium chloride.

5. A composition for use as claimed in any previous claim, **characterised in that** the nitric oxide releasing substance comprises L-arginine

6. A composition for use as claimed in any previous claim, **characterised in that** the nitric oxide releasing substance comprises L-arginine HCl.

7. A composition for use as claimed in any previous claim, **characterised in that** the nitric oxide releasing substance has a concentration of 0.25% to 25% w/v.

8. A composition for use as claimed in any previous claim, **characterised in that** the composition is contained within a topical delivery vehicle.

9. A composition for use as claimed in claim 2-3 **characterised in that** said composition is contained within a topical delivery vehicle and **in that** the topical delivery vehicle is hydrophobic.

10. A composition for use as claimed in claim 8 or 9, **characterised in that** the topical delivery vehicle is selected from the group consisting of topical creams, topical liquids, topical lotions and topical ointments.

11. A composition for use as claimed in any one of claims 2-3 **characterised in that** the topical delivery vehicle comprises water (20-80% w/v), mineral oil (3-18% w/v), glyceryl stearate (0.25-12% w/v), squalene (0.25-12% w/v), cetyl alcohol (0.1-11% w/v), propylene glycol stearate (0.1-11% w/v), wheat germ oil (0.1-6% w/v), polysorbate 60 (0.1-5% w/v), propylene glycol (0.05-5% w/v), collagen (0.05-5% w/v), sorbitan stearate (0.05-5% w/v), vitamin A&D (0.02-4% w/v), vitamin E (0.02-4% w/v), triethanolamine (0.01-4% w/v), methylparaben (0.01-4% w/v), aloe vera extract (0.01-4% w/v), imidazolidinyl urea (0.01-4% w/v), propylparaben (0.01-4% w/v), bha (0.01-4% w/v), L-arginine hydrochloride (0.25% to 25% w/v), and sodium chloride (0.25% to 25% w/v).

## Patentansprüche

1. Zusammensetzung zur Verwendung als Medikament zum topischen Aufbringen auf die Haut zur Schmerzbehandlung, wobei die Zusammensetzung Folgendes umfasst:
eine Stickoxid freisetzende Substanz, die aus einem Element der Gruppe ausgewählt ist, die aus L-Arginin, L-Argininsalzen und L-Argininderivaten besteht, welche aus der Gruppe ausgewählt sind, die aus Ethyl-, Methyl-, Propyl-, Isopropyl-, Butyl-, Isobutyl- oder t-Butylestern von L-Arginin und Salzen davon besteht, und
ein ionisches Salz, wobei das ionische Salz eine Ionenstärke von mindestens dem Zweifachen der physiologischen Ionenstärke von Blut aufweist.

2. Zusammensetzung zur Verwendung nach Anspruch 1, **dadurch gekennzeichnet, dass** die Zusammensetzung ferner Capsaicin umfasst.

3. Zusammensetzung zur Verwendung nach Anspruch 1, **dadurch gekennzeichnet, dass** die Zusammensetzung ferner Oleoresin capsicum umfasst.

4. Zusammensetzung zur Verwendung nach einem der vorhergehenden Ansprüche, **dadurch gekennzeichnet, dass** das ionische Salz Natriumchlorid umfasst.

5. Zusammensetzung zur Verwendung nach einem der vorhergehenden Ansprüche, **dadurch gekennzeichnet, dass** die Stickoxid freisetzende Substanz L-Arginin umfasst.

6. Zusammensetzung zur Verwendung nach einem der vorhergehenden Ansprüche, **dadurch gekennzeichnet, dass** die Stickoxid freisetzende Substanz L-Arginin-HCl umfasst.

7. Zusammensetzung zur Verwendung nach einem der vorhergehenden Ansprüche, **dadurch gekennzeichnet, dass** die Stickoxid freisetzende Substanz eine Konzentration von 0,25 % bis 25 %(w/v) aufweist.

8. Zusammensetzung zur Verwendung nach einem der vorhergehenden Ansprüche, **dadurch gekennzeichnet, dass** die Zusammensetzung in einem Vehikel zur topischen Abgabe enthalten ist.

9. Zusammensetzung zur Verwendung nach Anspruch 2 bis 3, **dadurch gekennzeichnet, dass** die Zusammensetzung in einem Vehikel zur topischen Abgabe enthalten ist und dass das Vehikel zur topischen Abgabe hydrophob ist.

10. Zusammensetzung zur Verwendung nach Anspruch 8 oder 9, **dadurch gekennzeichnet, dass** das Vehikel zur topischen Abgabe aus der Gruppe ausgewählt ist, die aus topischen Cremes, topischen Flüssigkeiten, topischen Lotionen und topischen Salben besteht.

11. Zusammensetzung zur Verwendung nach einem der Ansprüche 2 bis 3, **dadurch gekennzeichnet, dass** das Vehikel zur topischen Abgabe Wasser (20-80 %(w/v)), Mineralöl (3-18 %(w/v)), Glycerylstearat (0,25-12 %(w/v)), Squalen (0,25-12 %(w/v)), Cetylalkohol (0,1-11 %(w/v)), Propylenglycolstearat (0,1-11 %(w/v)), Weizenkeimöl (0,1-6 %(w/v)), Polysorbat 60 (0,1-5 %(w/v)), Propylenglycol (0,05-5 %(w/v)), Collagen (0,05-5 %(w/v)), Sorbitanstearat (0,05-5 %(w/v)), Vitamin A und D (0,02-4 %(w/v)), Vitamin E (0,02-4 %(w/v)), Triethanolamin (0,01-4 %(w/v)), Methylparaben (0,01-4 %(w/v)) Aloe-vera-Extrakt (0,01-4 %(w/v)), Imidazolidinylharnstoff (0,01-4 %(w/v)), Propylparaben (0,01-4 %(w/v)), BHA (0,01-4 %(w/v)), L-Arginin-Hydrochlorid (0,25 bis 25 %(w/v)) und Natriumchlorid (0,25 bis 25 %(w/v)) umfasst.

## Revendications

1. Composition pour une utilisation en tant que médicament en application topique sur la peau pour le traitement de la douleur, la composition comprenant :
une substance libérant du monoxyde d'azote sélectionnée dans le groupe consistant en la L-arginine, des sels de la L-arginine et des dérivés de la L-arginine sélectionnés dans le groupe consistant en les esters éthylique, méthylique, propylique, isopropylique, butylique, isobutylique ou t-butylique de la L-arginine et les sels de ceux-ci ; et
un sel ionique, le sel ionique ayant une force ionique qui correspond au double au moins de celle de la force ionique physiologique du sang.

2. Composition pour l'utilisation selon la revendication 1, **caractérisée en ce que** la composition comprend également de la capsaïcine.

3. Composition pour l'utilisation selon la revendication 1, **caractérisée en ce que** la composition comprend également de l'oléorésine de capsicum.

4. Composition pour l'utilisation selon l'une quelconque des revendications précédentes, **caractérisée en ce que** le sel ionique comprend le chlorure de sodium.

5. Composition pour l'utilisation selon l'une quelconque des revendications précédentes, **caractérisée en ce que** la substance libérant du monoxyde d'azote comprend la L-arginine.

6. Composition pour l'utilisation selon l'une quelconque des revendications précédentes, **caractérisée en ce que** la substance libérant du monoxyde d'azote comprend la L-arginine HCl.

7. Composition pour l'utilisation selon l'une quelconque des revendications précédentes, **caractérisée en ce que** la substance libérant du monoxyde d'azote a une concentration allant de 0,25 % à 25 % p/v.

8. Composition pour l'utilisation selon l'une quelconque des revendications précédentes, **caractérisée en ce que** la composition est contenue dans un véhicule de libération topique.

9. Composition pour l'utilisation selon les revendications 2-3, **caractérisée en ce que** ladite composition est contenue dans un véhicule de libération topique et **en ce que** le véhicule de libération topique est hydrophobe.

10. Composition pour l'utilisation selon les revendications 8 ou 9, **caractérisée en ce que** le véhicule de libération topique est sélectionné dans le groupe consistant en des crèmes à usage topique, des liquides à usage topique, des lotions à usage topique et des pommades à usage topique.

11. Composition pour l'utilisation selon les revendications 2-3, **caractérisée en ce que** le véhicule de libération topique comprend les composants suivants : eau (20-80 % p/v), huile minérale (3-18 % p/v), stéarate de glycéryle (0,25-12 % p/v), squalène (0,25-12 % p/v), alcool cétylique (0,1-11 % p/v), stéarate de propylène-glycol (0,1-11 % p/v), huile de germes de blé (0,1-6 % p/v), polysorbate 60 (0,1-5 % p/v), propylène-glycol (0,05-5 % p/v), collagène (0,05-5 % p/v), stéarate de sorbitane (0,05-5 % p/v), vitamines A & D (0,02-4 % p/v), vitamine E (0,02-4 % p/v), triéthanolamine (0,01-4 % p/v), parabène méthylique (0,01-4 % p/v), extrait d'Aloe vera (0,01-4 % p/v), imidazolidinylurée (0,01-4 % p/v), parabène propylique (0,01-4 % p/v), BHA (0,01-4 % p/v), hydrochlorure de L-arginine (0,25-25 % p/v) et chlorure de sodium (0,25-25 % p/v).
